Europäisches Patentamt

European Patent Office

Office européen des brevets

(19)

(11) Numéro de publication: **0 361 610 B1**

## (12) FASCICULE DE BREVET EUROPEEN

(45) Date de publication de fascicule du brevet: **03.08.94**  (51) Int. Cl.5: **G01S 15/58**, A61B 8/06

(21) Numéro de dépôt: **89202407.6**

(22) Date de dépôt: **25.09.89**

(54) **Dispositif de mesure par échographie de la vitesse transverse d'organes en mouvement et d'écoulements sanguins.**

(30) Priorité: **30.09.88 FR 8812806**

(43) Date de publication de la demande:
**04.04.90 Bulletin  90/14**

(45) Mention de la délivrance du brevet:
**03.08.94 Bulletin  94/31**

(84) Etats contractants désignés:
**DE FR GB**

(56) Documents cités:
**EP-A- 0 010 974**
**EP-A- 0 177 942**
**EP-A- 0 225 667**
**DE-A- 3 545 470**

**ELECTRONICS LETTERS, vol. 24, no. 4, 18 février 1988, pages 205-207, Stevenage,Herts, GB; M. NIKOONAHAD et al.: "High-resolution ultrasound transverse flow measurement"**

(73) Titulaire: **LABORATOIRES D'ELECTRONIOUE PHILIPS**
**3, Avenue Descartes**
**F-94450 Limeil-Brévannes(FR)**
(84) Etats contractants désignés:
**FR**

(73) Titulaire: **N.V. Philips' Gloeilampenfabrieken**
**Groenewoudseweg 1**
**NL-5621 BA Eindhoven(NL)**
(84) Etats contractants désignés:
**DE GB**

(72) Inventeur: **Bonnefous, Odile Société Civile S.P.I.D.**
**209, rue de l'Université**
**F-75007 Paris(FR)**

(74) Mandataire: **Charpail, François**
**Société Civile S.P.I.D.**
**156, Boulevard Haussmann**
**F-75008 Paris (FR)**

EP 0 361 610 B1

**Description**

La présente invention concerne un dispositif de mesure, par échographie ultrasonore, de la vitesse d'organes en mouvement et d'écoulements sanguins, la composante axiale Vz de la vitesse, parallèle à l'axe Oz du faisceau ultrasonore d'exploration, étant mesurée à l'aide d'une part, d'un premier circuit d'intercorrélation fonctionnant à un pas d'échantillonnage $\Delta T$, et délivrant, à partir de deux lignes échographiques successives décalées de $k\Delta T$ ($k = -I, -I+1,...,I$), $2I+1$ valeurs de fonctions de corrélation suivant z, la corrélation étant faite entre les signaux ultrasonores successifs et décalés par les intervalles d'échantillonnage $k\Delta T$($k = -I, -I+1,...,I$), et d'autre part, d'un premier circuit de recherche de maximum, donnant la valeur $I_o$ de k correspondant à la valeur la plus grande desdites fonctions de corrélation suivant z.

L'invention trouve une application particulièrement avantageuse dans le domaine de l'exploration échographique d'organes en mouvement, tels que les parois du coeur, et d'écoulements sanguins dans les vaisseaux.

Actuellement, les vélocimètres Doppler habituellement utilisés ne permettent de mesurer que les vitesses axiales des mouvements étudiés. Cette mesure peut être réalisée par différentes méthodes de traitement des signaux échographiques, et notamment la méthode d'intercorrélation décrite dans la demande de brevet francais n° 2 590 790. Cette méthode connue utilise le fait que les signaux ultrasonores successifs rétrodiffusés par une cible en mouvement vers le transducteur ultrasonore sont reliés par l'équation suivante :

$$S_{n+1}(t) = S_n(t-\tau_z)$$

$\tau_z$ étant donné par :

$$\tau_z = 2V_z T/C$$

où T est la période de récurrence et C la vitesse du son.

La fonction d'intercorrélation entre $S_n(t)$ et $S_{n+1}(t)$ est défini par :

$$\int S_{n+1}(t+u)S_n(t)dt = C_{nn}(u-\tau_z)$$

La fonction $C_{nn}(u-\tau_z)$ est une fonction d'autocorrélation et, de ce fait, est maximale pour $u = \tau_z$. Ainsi, une mesure du décalage temporel $\tau_z$, et donc de la vitesse axiale $V_z$, peut se faire en cherchant pour quel paramètre u la fonction d'intercorrélation est maximale. A cet effet, la fonction d'intercorrélation est échantillonnée, à un pas d'échantillonnage $\Delta T$, entre $-I\Delta T$ et $+I\Delta T$ par pas de 1 de façon à obtenir $2I+1$ valeurs de fonctions de corrélation. La valeur maximum de ces $2I+1$ valeurs correspondant à $u = Io\Delta T$ permet de mesurer la vitesse axiale $V_z$ en utilisant l'égalité :

$$V_z = IoC\Delta T/2T$$

La demande de brevet français n° 2 590 790 enseigne également qu', afin de s'affranchir des erreurs inhérentes à l'échantillonnage dans la détermination du maximum de la fonction de corrélation, il est tout à fait avantageux d'utiliser un circuit d'interpolation fournissant à partir des valeurs de fonctions de corrélation une estimée plus précise de la vitesse axiale.

Toutefois, ce dispositif de mesure connu n'apporte aucune information relative aux composantes transverses $V_x$ et $V_y$ de la vitesse du mouvement ou de l'écoulement étudié. Or ces deux paramètres seraient d'une importance majeure pour déterminer, par exemple, le débit sanguin et pour obtenir une meilleure visualisation des écoulement sanguins.

Aussi, le problème technique à résoudre par l'objet de la présente invention est de réaliser un dispositif de mesure par échographie ultrasonore, non seulement de la composante axiale $V_z$, mais aussi des composantes transverses $V_x$, $V_y$ de la vitesse d'organes en mouvement et d'écoulements sanguins.

Une solution au problème technique posé consiste, selon la présente invention, en ce que ledit dispositif comporte :

- une mosaïque bidimensionnelle de transducteurs piézoélectriques, centrée en l'origine O d'un système d'axes $O_x$, $O_y$ formant trièdre avec ledit axe $O_z$,
- un circuit de formation de $2(N_x + N_y) + 1$ voies en réception correspondant à des faisceaux parallèles à l'axe $O_z$ et passant par les points suivants du plan $O_x$, $O_y$ :
d'une part, les points $(k_x p_x, O, O)$, avec $k_x = -N_x, -N_x+1,...,N_x$ et $p_x$ le pas des voies selon x, et, d'autre part, les points $(O, k_y p_y, O)$, avec $k_y = -N_y, -N_y+1,...,N_y$ et $p_y$ le pas des voies selon y,
- une mémoire-tampon où sont stockés les signaux échographiques reçus par chacune des $2(N_x + N_y) + 1$ voies en réception, pris pour les valeurs $I_o-1$, $I_o$, $I_o+1$ de k,
- un deuxième, respectivement un troisième, circuit d'intercorrélation entre un signal $S_n$-$(O,O,t)$ reçu sur la voie de réception centrale $(O,O,O)$ et le signal suivant $S_{n+1}(k_x p_x, O, t + m\Delta T)$ reçu sur la voie de réception $(k_x p_x, O, O)$, respectivement le signal suivant $S_{n+1}(O, k_y p_y, t + m\Delta T)$ reçu sur la voie de réception $(O, k_y p_y, O)$ avec $m = I_o-1, I_o, I_o+1$, donnant les 3 fonctions d'intercorrélation $C_x$-

$(k_x p_x, O, (I_O-1)\Delta T)$, $C_x(k_x p_x, O, I_O \Delta T)$, $C_x(k_x p_x, O, -(I_O+1)\Delta T)$, respectivement $C_y(O, k_y p_y, (I_O-1)-\Delta T)$, $C_y(O, k_y p_y, I_O \Delta T)$, $C_y(O, k_y p_y, (I_O+1)\Delta T)$,

- un circuit d'interpolation linéaire en x, respectivement y, fournissant à partir desdites 3 fonctions d'intercorrélation le pic de corrélation $C_x(k_x p_x)$ selon x, respectivement $C_y(k_y p_y)$ selon y,
- un deuxième, respectivement un troisième circuit de recherche de maximum, donnant, à partir des $2N_x+1$ valeurs de $k_x$, respectivement les $2N_y+1$ valeurs de $k_y$, la valeur $k_{xo}$, respectivement $k_{yo}$, pour laquelle $C_x(k_x p_x)$, respectivement $C_y(k_y p_y)$, est maximum, les composantes transverses $V_x$ et $V_y$ vérifiant les égalités :

$$V_x = k_{xo}p_x/T$$
$$V_y = k_{yo}p_y/T$$

T étant la période de récurrence des signaux échographiques.

Soit un diffuseur animé d'une vitesse $\vec{v}$, de composantes $V_x$, $V_y$ et $V_z$, situé dans le trièdre Oxyz au point de coordonnées x, y, z, z étant la profondeur d'exploration. Le nième signal échographique reçu par la voie centrée au point (O,O,O) de la mosaïque est donné par :

$$S_n(O,O,t) = \int\int p(x,y,t) D_n(x,y)\, dxdy$$

$p(x,y,t)$ étant la réponse de diffraction du faisceau et $D_n(x,y)$ la réponse de diffusion du milieu à l'instant nT. $D_n(x,y)$ vérifie la relation de récurrence suivante.

$$D_{n+1}(x,y) = D_n(x-V_x T, y-V_y T)$$

ce qui donne :

$$S_{n+1}(x,y,t) = S_n(x-V_x T, y-V_y T, t-2V_z T/C)$$

La fonction d'intercorrélation des signaux $S_n$-(O,O,t) et $S_{n+1}$(O,O,t) est fournie par l'expression :

$$C_{nn+1}(O,O,u) = \int S_n(O,O,t)S_{n+1}(O,O,t+u)dt$$
$$= \int S_n(O,O,t)S_n(-V_x T, -V_y T, t-2V_z T/C+u)d$$
$$= C_{nn}(-V_x T, -V_y T, u-2V_z T/C)$$

Par rapport à la variable u, $C_{nn}$ est une fonction d'autocorrélation qui prend une valeur maximum pour $u=2V_z T/C$. On retrouve ainsi les mêmes considérations qui ont conduit à la méthode de mesure de la vitesse axiale $V_z$ rappelée plus haut, à savoir échantillonnage sur $2I+1$ échantillons de pas $\Delta T$, et recherche de la valeur $I_o$ donnant la plus grande valeur à la fonction d'intercorrélation.

Considérons maintenant le nième signal échographique reçu sur la voie centrée au point $(k_x p_x, O, O)$ de la mosaïque :

$$S_n(k_x p_x, O, t) = \int\int p(x-k_x p_x, y, t) D_n(x,y)dt$$

La fonction d'intercorrélation des signaux $S_n$-(O,O,t) et $S_{n+1}(k_x p_x, O, t)$ est donnée par :

$$C_{nn+1}(k_x p_x, O, u) = \int S_n(O,O,t)S_{n+1}(k_x p_x, O, t+u)dt$$
$$= \int S_n(O,O,t)S_n(k_x p_x - V_x T, -V_y T, t-2V_z T/C+u)dt$$
$$= C_{nn}(k_x p_x - V_x T, -V_y T, u-2V_z T/C)$$

De façon à augmenter la sensibilité de la mesure, on peut, si on le désire, cumuler, dans un accumulateur, les valeurs de la fonction d'intercorrélation entre deux signaux consécutifs n et n + 1.

$$C_x(k_x p_x, O, u) = \Sigma_n C_n(k_x p_x - V_x T, -V_y T, u-2V_z T/C)$$

Cette fonction de corrélation en x est maximum pour $u=2V_z T/C$.

Son maximum $C_x(k_x p_x, O, 2V_z T/C)$ est déterminé par interpolation linéaire sur 3 valeurs échantillonnées par rapport à u, prises autour de $I_o \Delta T$, soit $(I_o-1)\Delta T$, $I_o \Delta T(I_o+1)\Delta T$. La fonction $C_x$-$(k_x p_x, O, 2V_z T/C) = C_x(p_x k_x)$ est une fonction d'autocorrélation en x, et est, de ce fait, maximum pour $k_x p_x - V_x T = O$. Par échantillonnage sur les $2N_{x+1}$ valeurs de $k_x$ et recherche de maximum, on peut déterminer celle, $k_{xo}$, qui donne la plus grande valeur de $C_x(k_x p_x)$. Alors la vitesse transverse selon x est égale à

$$V_x = k_{xo}p_x/T.$$

On montre de la même manière que la vitesse transverse selon y est donnée par :

$$V_y = k_{yo}p_y/T.$$

$k_{yo}$ correspondant au maximum de la fonction d'intercorrélation suivant y

$C_y(p_y k_y) = C_y(O, k_y p_y, 2V_z T/C)$ défini par
$$C_y(O, k_y p_y, u) = \Sigma_n C_{nn}(-V_x T, k_y p_y - V_y T, u-2V_z T/C) = \Sigma_n \int S_n(O,O,t)S_{nn}(O, k_y p_y, t+u)dt$$

Comme pour la vitesse axiale $V_z$, on peut obtenir une estimée plus précise des composantes transverses $V_x$, $V_y$ qui ne soit pas affectée par les erreurs dues à l'échantillonnage. Par cela, il est prévu que lesdits deuxième et troisième circuits de recherche de maximum sont suivis d'un premier, respectivement un deuxième, circuit d'interpolation donnant la valeur $k_{xmax}$, respectivement $k_{ymax}$, pour laquelle la fonction d'intercorrélation $C_x(k_x p_x)$, res-

pectivement $C_y(k_y p_y)$ est maximum, les composantes transverses vérifiant les égalités :

$$V_x = k_{xmax} p_x/T$$
$$V_y = k_{ymax} p_y/T$$

Pour des raisons de simplicité de réalisation, on envisage que les premier, deuxième et troisième circuits d'intercorrélation sont des corrélateurs 1 bit, en ce sens que les signaux $S_n$ et $S_{n+1}$ utilisés sont réduits au signe du signal ultrasonore. Dans ce cas, le pic de la fonction de corrélation est de forme triangle isocèle. Aussi peut-on prévoir que les premier et deuxième circuits d'interpolation effectuent une interpolation linéaire à partir du point le plus haut et de ses deux voisins de façon à reconstituer complètement le pic de corrélation et donc de déterminer avec précision les valeurs de $k_{xmax}$ et $k_{ymax}$.

Enfin, il faut noter que, pour des vitesses transverses $V_{x,y}$ de l'ordre de 50 cm/s et une fréquence de récurrence T de 200 μs, la quantité $V_{x,y}T$ vaut environ 0,1 mm. Aussi, le pas $p_x$ ou $p_y$ entre deux faisceaux consécutifs devrait avoir une valeur équivalente. Or, en pratique, pour une barrette linéaire de transducteurs fonctionnant à 5MHz, le pas entre faisceaux est voisin de 0,3 mm. Il est bien sûr possible d'imaginer de réaliser une barrette de 0,1 mm de pas, toutefois, cela exigerait une électronique de formation de faisceaux complexe et impliquerait des modules de forte impédance incompatible avec un faible bruit en réception. C'est pour éviter cet inconvénient qu'il y a avantage à ce qu'un interpolateur transverse, placé avant ladite mémoire tampon, effectue une réduction d'un facteur n sur les pas $p_x$ et $p_y$. Dans l'exemple numérique ci-dessus, le facteur n est égal à 3. Cependant, dans l'ensemble de ce mémoire, on appellera, sans distinction, $p_x$ et $p_y$ indifférement le pas réel ou le pas fictif (divisé par n) des faisceaux selon x et y.

La description qui va suivre en regard des dessins annexés, donnés à titre d'exemples non limitatifs, fera bien comprendre en quoi consiste l'invention et comment elle peut être réalisée.

La figure 1 est le schéma d'un dispositif selon l'invention jusqu'à la mémoire-tampon.

La figure 2 est le schéma de la voie de traitement de la vitesse axiale.

La figure 3 est le schéma de la voie de traitement de la vitesse transverse.

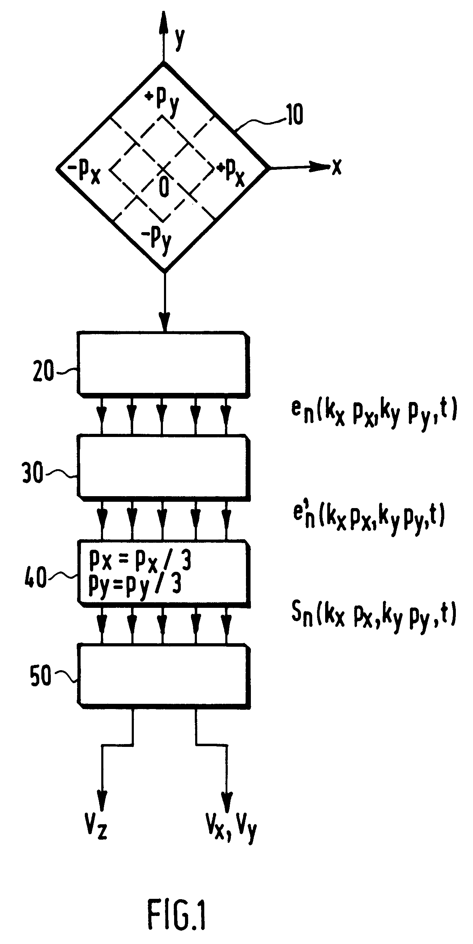

La figure 1 montre le schéma d'un dispositif de mesure, par échographie ultrasonore, des composantes $V_x, V_y$ de la vitesse d'organes en mouvement et d'écoulements sanguins. Ce dispositif comporte une mosaïque bidimensionnelle 10 de transducteurs piézoélectriques, centrée en l'origine O d'un système d'axes $O_x$ et $O_y$ formant trièdre avec un axe z, non représenté, perpendiculaire au plan de la figure, $O_z$ définissant la direction des faisceaux ultrasonores d'exploration. Un circuit 20 de formation de voies réalise $2(N_x + N_y) + 1 = 5$ voies en réception (on a pris ici $N_x = N_y = 1$), correspondant à des faisceaux centrés en $(k_x p_x, O, O)$ avec $k_x = -1, 0, 1$ et en $(O, k_y p_y, O)$ avec $k_y = -1, 0, 1$. Les 5 faisceaux ainsi définis sont déterminés par les 5 zones de la mosaïque 10 représentées à la figure 1. Dans l'exemple montré, ces zones sont carrées puisqu'on a pris ici les pas $p_x$ et $p_y$ égaux. Les 5 voies $e_n(k_x p_x, k_y p_y, t)$ sont ensuite traitées de façon classique par un circuit 30 d'élimination d'échos fixes. Les signaux $e'_n(k_x p_x, k_y p_y, t)$ ainsi obtenus subissent, si nécessaire, une affinité de pas, d'un facteur 3 par exemple, à l'aide d'un interpolateur linéaire 40 délivrant en sortie les signaux $e''_n$ donnés par

$$e''_n(k_x p_x/3, k_y p_y/3, t) = 1/3 e'_n(k_x p_x, k_y p_y, t) + 2/3 e'_n(O, O, t)$$
$$= S_n(k_x p_x, k_y p_y, t)$$

Les signaux $S_n(k_x p_x, k_y p_y, t)$ sont alors stockés dans une mémoire-tampon 50.

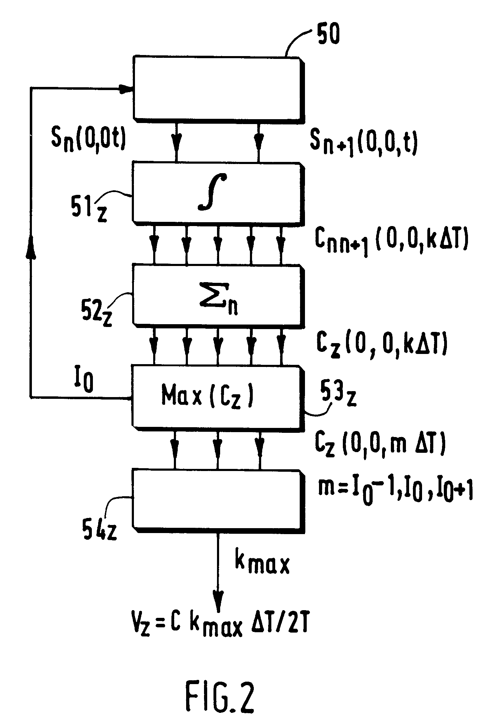

La figure 2 montre la voie de traitement qui aboutit à la détermination de la vitesse axiale $V_z$. Les signaux $S_n(O, O, t)$ et $S_{n+1}(O, O, t)$, extraits de la mémoire-tampon 50, sont corrélés par le corrélateur $51_z$, par exemple du type "1 bit", et échantillonnés sur $2l + 1$ pas d'échantillonnage $\Delta T$. Pour chaque échantillon $k = -l, -l+1, ..., l$, les fonctions d'intercorrélation $C_{nn+1}(O, O, k\Delta T)$ sont sommées sur n dans un accumulateur $52_z$. Puis, un circuit $53_z$ de recherche de maximum donne la valeur $l_o$ de l'échantillon correspondant à la valeur la plus grande de la fonction de corrélation $C_z(O, O, k\Delta T)$. Cette valeur $l_o$ est stockée dans la mémoire-tampon 50 pour utilisation ultérieure dans la mesure de la vitesse transverse. Une valeur approchée de la vitesse axiale $V_z$ est donnée par $V_z = C l_o \Delta T/2T$. De façon à obtenir une valeur plus précise de $V_z$, on peut effectuer une interpolation linéaire sur les 3 valeurs de la fonction de corrélation pour $l_o-1, l_o+1$ afin d'en trouver le maximum absolu et la valeur correspondante de k soit $k_{max}$, $V_z$ vaut alors $C k_{max} \Delta T/2T$.

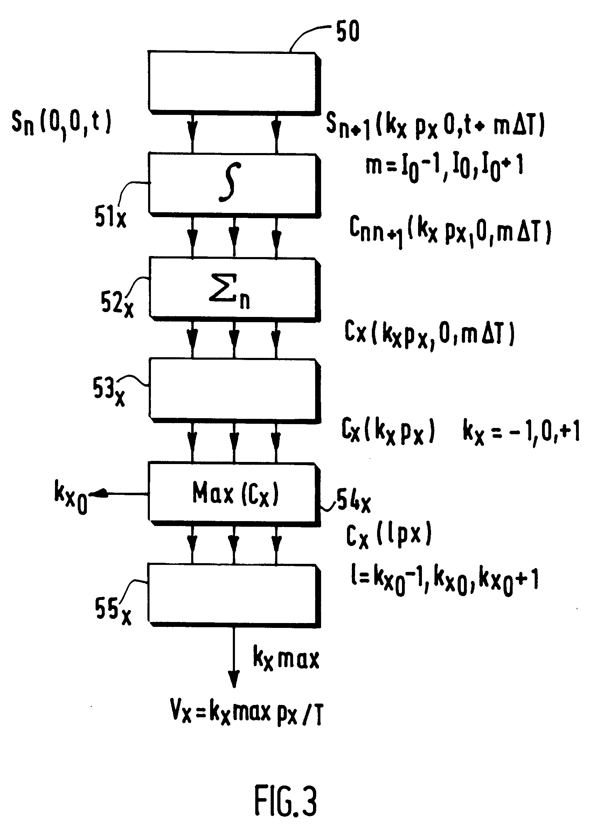

La figure 3 est le schéma de la voie de traitement qui conduit à la mesure de la composante $V_x$ de la vitesse transverse, étant entendu qu'un traitement analogue en y permet de la même façon de déterminer la composante $V_y$.

A partir des signaux $S_n(O, O, t)$, d'une part, et $S_{n+1}(k_x p_x, O, t + m\Delta T)$ avec $m = l_o-1, l_o, l_o+1$, le corrélateur $51_x$, "1 bit" par exemple, construit les 3 fonctions de corrélation $C_{nn+1}(k_x p_x, O, m\Delta T)$ qui, une fois sommées par l'accumulateur $52_x$, fournissent les fonctions d'intercorrélation $C_x(k_x p_x, O, m\Delta T)$. Un circuit $53_x$ d'interpolation linéaire calcule ensuite le

pic de corrélation $C_x(k_x p_x)$ correspondant au maximum de $C_x(k_x p_x, O, u)$ pour u compris entre $(I_o-1)\Delta T$ et $(I_o+1)\Delta T$.

Un circuit $54_x$ de recherche de maximum détermine la valeur $k_{xo}$ de $k_x$ donnant la valeur la plus grande du pic de corrélation $C_x(k_x p_x)$; une valeur approchée de la vitesse transverse $V_x$ est alors fournie par :

$$V_x = k_{xo}p_x/T.$$

Pour avoir une précision plus grande sur $V_x$, on effectue une interpolation linéaire sur les 3 valeurs $C_x((k_{xo}-1)p_x)$, $C_x(k_o p_x)$, $C_x((k_o+1)p_x)$ du pic de corrélation de façon à déterminer $k_{xmax}$ correspondant au maximum du pic de corrélation, $V_x$ vaut alors

$$V_x = k_{xmax}p_x/T.$$

## Revendications

1. Dispositif de mesure, par échographie ultrasonore, de la vitesse d'organes en mouvement et d'écoulements sanguins, la composante axiale $V_z$ de la vitesse, parallèle à l'axe $O_z$ du faisceau ultrasonore d'exploration, étant mesurée à l'aide d'une part, d'un premier circuit d'intercorrélation ($51_z$) fonctionnant à un pas d'échantillonnage $\Delta T$, et délivrant, à partir de deux lignes échographiques successives décalées de $k\Delta T(k = -I, -I+1, ...I)$, $2I+1$ valeurs de fonctions de corrélation suivant z, la corrélation étant faite entre les signaux ultrasonores successifs et décalés par les intervalles. d'échantillonnage $k\Delta T(k = -I, -I+1, ..., I)$, et d'autre part, d'un premier circuit ($53_z$) de recherche de maximum, donnant la valeur $I_o$ de k correspondant à la valeur la plus grande desdites fonctions de corrélation suivant z, caractérisé en ce que ledit dispositif comporte pour mesurer les composantes transverses $V_x$, $V_y$ de la vitesse :
    - une mosaïque bidimensionnelle (10) de transducteurs piézoélectriques, centrée en l'origine O d'un système d'axes $O_x$, $O_y$ formant trièdre avec ledit axe $O_z$,
    - un circuit (20) de formation de $2(N_x + N_y)+1$ voies en réception correspondant à des faisceaux parallèles à l'axe $O_z$ et passant par les points suivants du plan $O_x$, $O_y$ : d'une part, les points $(k_x p_x, O, O)$, avec $k_x = -N_x, -N_x+1, ..., N_x$ et $p_x$ le pas des voies selon x, et, d'autre part, les points $(O, k_y p_y, O)$ avec $k_y = -N_y, -N_y+1, ..., N_y$ et $p_y$ le pas des voies selon y,
    - une mémoire-tampon (50) où sont stockés les signaux échographiques reçus par chacune des $2(N_x+N_y)+1$ voies en réception, pris pour les valeurs $I_o-1$, $I_o$ et $I_o+1$ de k,
    - un deuxième ($51_x$), et respectivement un troisième ($51_y$), circuit d'intercorrélation entre un signal $S_n(O,O,t)$ reçu sur la voie de réception centrale $(O,O,O)$ et le signal suivant $S_n + 1$ $(k_x p_x, O, t+m\Delta t)$ reçu sur la voie de réception $(k_x p_x, O, O)$, respectivement le signal suivant $S_n + 1(O, k_y p_y, t + m\Delta T)$ reçu sur la voie de réception $(O, k_y p_y, O)$, avec $m = I_0-1, I_0, I_0+1$, donnant les 3 fonctions d'intercorrelation $C_x(k_x p_x, O, (I_o-1)\Delta T)$, $C_x(k_x p_x, O, I_o \Delta T)$, $C_x(k_x p_x, O, (I_o+1)\Delta T)$ respectivement $C_y(O, k_y p_y, (I_o-1)\Delta T)$, $C_y(O, k_y p_y, I_o \Delta T)$, $C_y(O, k_y p_y, (I_o+1)\Delta T)$,
    - un circuit ($53_x$) d'interpolation linéaire en x, respectivement en y ($53_y$), fournissant à partir desdites 3 fonctions d'intercorrélation le pic de corrélation $C_x(k_x p_x)$ selon x, respectivement $C_y(k_y p_y)$ selon y,
    - un deuxième ($54_x$), respectivement un troisième ($54_y$), circuit de recherche de maximum, donnant, à partir des $2N_x + 1$ valeurs de $k_x$, respectivement des $2Ny + 1$ valeurs de $k_y$, la valeur $k_{xo}$, respectivement $k_{yo}$, pour laquelle $c_x(k_x p_x)$, respectivement $C_y(k_y p_y)$, est maximum, les composantes transverses $V_x$ et $V_y$ vérifiant les égalités :

$$V_x = k_{xo}p_x/T$$
$$V_y = k_{yo}p_y/T$$

T étant la période de récurrence des signaux échographiques.

2. Dispositif selon la revendication 1, caractérisé en ce que lesdits deuxième ($54_x$) et troisième ($54_y$) circuits de recherche de maximum sont suivis d'un premier ($55_x$), respectivement un deuxième ($55_y$), circuit d'interpolation donnant la valeur $k_{xmax}$, respectivement $k_{ymax}$, pour laquelle la fonction d'intercorrélation $C_x(k_x p_x)$, respectivement $C_y(k_y p_y)$, est maximum, les composantes transverses vérifiant les égalités :

$$V_x = k_{xmax}p_x/T$$
$$V_y = k_{ymax}p_y/T$$

3. Dispositif selon la revendication 1 ou 2 caractérisé en ce que les premier ($51_z$), deuxième ($51_x$) et troisième ($51_y$) circuits d'intercorrélation sont des corrélateurs 1 bit.

4. Dispositif selon les revendications 2 et 3, caractérisé en ce que les premier ($54_x$) et deuxième ($54_y$) circuits d'interpolation effectuent une interpolation linéaire.

5. Dispositif selon l'une quelconque des revendications 1 à 4, caractérisé en ce qu'un interpolateur transverse (40), placé avant ladite mémoire-tampon (50), effectue une réduction d'un facteur n sur les pas $p_x$ et $p_y$.

6. Dispositif selon l'une quelconque des revendications 1 à 5, caractérisé en ce que le circuit (20) de formation de voies est suivi d'un circuit (30) d'élimination d'échos fixes.

**Claims**

1. A device for measuring the speed of moving organs and blood flows by means of ultrasonic echography, the axial component $V_z$ of the speed, oriented parallel to the axis $0_z$ of the ultrasonic scanning beam, being measured by means of on the one hand a first intercorrelation circuit ($51_z$) which operates with a sampling step $\Delta T$ and which supplies, on the basis of two successive echographic lines shifted through $k\Delta T$ ($k$ = -I, -I+1, ..., I), 2I+1 correlation function values according to z, the correlation being established between the successive ultrasonic signals shifted through the sampling intervals $k\Delta T$ ($k$ = -I, -I+1, ..., I), and on the other hand by means of a first maximum-value searching circuit ($53_z$) which supplies the value $I_o$ of k which corresponds to the largest value of said correlation functions according to z, characterized in that for the measurement of the transverse components $V_x$, $V_y$ of the speed said device comprises:

- a bidimensional mosaic (10) of piezoelectric transducers which are centred around the origin 0 of a system of yes $0_x$, $0_y$ which form a trihedron with said axis $0_z$,
- a circuit (20) for forming $2(N_x + N_y) + 1$ receiving channels which correspond to beams parallel to the axis $0_z$ and passing through the following points of the plane $0_x$, $0_y$: on the one hand the points $(k_xp_x,0,0)$, where $k_x$ = -$N_x$, -$N_x$+1, ..., $N_x$ and $p_x$ is the pitch of the channels according to x and, on the other hand, the points $(0,k_yp_y,0)$, where $k_y$ = -$N_y$, -$N_y$+1, ..., $N_y$ and $P_y$ is the pitch of the channels according to y,
- a buffer memory (50) in which there are stored the echographic signals received by each of the 2 $(N_x + N_y) + 1$ receiving channels, taken for the values $I_o$-1, $I_o$, $I_o$+1 of k,

- a second ($51_x$) and a third ($51_y$) circuit for intercorrelation between a signal ($S_n$-(0,0,t)) received by the central receiving channel (0,0,0) and the next signal $S_{n+1}$ - ($K_xP_x$,0,t+m$\Delta$t) received by the receiving channel ($k_xp_x$,0,0) and the next signal $S_{n+1}$ (0,$K_yP_y$,t+m$\Delta$T) received by the receiving channel (0,$K_y$,$P_y$,0), where m = $I_o$-I, $I_o$,$I_o$+1 giving the 3 intercorrelation functions
$C_x(k_xp_x,0,(I_o-1)\Delta T)$, $C_x(k_xp_x,0,I_o\Delta T)$, $C_x(k_xp_x,0,(I_o+1)\Delta T)$ and
$C_y(0,k_yp_y,(I_o-1)\Delta T)$, $C_y(0,k_yp_y,I_o\Delta T)$, $C_y(0,k_yp_y,(I_o+1)\Delta T$, respectively,
- a circuit for linear interpolation in x ($53_x$) and y ($53_y$), respectively, supplying the correlation peak $C_x(k_xp_x)$ according to x and $C_y(k_yp_y)$ according to y, respectively, on the basis of said 3 intercorrelation functions,
- a second ($54_x$) and a third ($54_y$) maximum-value searching circuit which supply, on the basis of the $2N_x + 1$ values of $k_x$ and the $2N_y + 1$ values of $k_y$, the values $k_{x0}$ and $k_{y0}$, respectively, for which $C_x(k_xp_x)$ and $C_y(k_yp_y)$, respectively, are maximum, the transverse components $V_x$ and $V_y$ satisfying the equations:

$$V_x = k_{x0}p_x/T$$
$$V_y = k_{y0}p_y/T$$

where T is the recurrent period of the echographic signals.

2. A device as claimed in Claim 1, characterized in that said second ($54_x$) and third ($54_y$) maximum-value searching circuits are followed by a first ($55_x$) and a second ($55_y$) interpolation circuit, respectively, supplying the value $k_{xmax}$ and $k_{ymax}$, respectively, for which the intercorrelation function $C_x(k_xp_x)$ and $C_y(k_yp_y)$, respectively, is maximum, the transverse components satisfying the equations:

$$V_x = k_{xmax}p_x/T$$
$$V_y = k_{ymax}p_y/T.$$

3. A device as claimed in Claim 1 or 2, characterized in that the first ($51_z$), the second ($51_y$) intercorrelation circuit are formed by 1-bit correlators.

4. A device as claimed in the Claims 2 and 3, characterized in that the first ($54_x$) and the second ($54_y$) interpolation circuit perform a linear interpolation.

5. A device as claimed in any one of the Claims 1 to 4, characterized in that a transverse interpolator (40) which precedes said buffer memory (50) reduces the pitches $p_x$ and $p_y$ by a factor n.

6. A device as claimed in any one of the Claims 1 to 5, characterized in that the circuit (20) for forming channels is followed by a circuit (30) for eliminating fixed echos.

**Patentansprüche**

1. Ultraschall-Echographiegerät zum Messen der Geschwindigkeit von bewegten Organen und der Fließgeschwindigkeit von Blut, wobei die parallel zur Achse $0_z$ des Ultraschallstrahls verlaufende Axialkomponente $V_z$ der Geschwindigkeit einerseits mit Hilfe einer ersten Interkorrelationsschaltung ($51_z$) mit einer Abtastfrequenz $\Delta T$, die ausgehend von zwei im Abstand von $k\Delta T$ ($k = -l, -l+1, ..., l$) erzeugten Echographiekurven, $2l+1$ aufeinanderfolgende Werte der Korrelationsfunktionen nach z liefert, wobei die Korrelation zwischen den in den Abtastintervallen $k\Delta T$ ($= -l, -l+1, ..., l$) aufeinanderfolgenden Ultraschallsignalen hergestellt wird, und andererseits mit Hilfe einer ersten Hochstwertsuchschaltung ($53_z$) gemessen wird, die den Wert $l_0$ von k liefert, der dem größten Wert der genannten Korrelationsfunktionen nach z entspricht,
dadurch gekennzeichnet, daß das genannte Gerät zum Messen der transversalen Komponenten $V_x$, $V_y$ der Geschwindigkeit folgendes umfaßt:
   - ein zweidimensionales Mosaik (10) aus piezoelektrischen Wandlern, dessen Mittelpunkt im Nullpunkt 0 eines Achsenkreuzes $0_x$, $0_y$ liegt, das mit der genannten Achse $0_z$ einen Dreiflächer bildet,
   - eine Erzeugerschaltung (20) mit $2 \cdot (N_x + N_y) + 1$ Empfangskanälen entsprechend den zur Achse $0_z$ parallelen und durch die folgenden Punkte der Ebene $0_x$, $0_y$ gehenden Strahlen: einerseits die Punkte ($k_x p_x, 0, 0$) mit $k_x = -N_x, -N_x + 1, ..., N_x$ und $p_x$ dem Kanalabstand in x, und andererseits die Punkte ($0, k_y p_y, 0$) mit $k_y = -N_y + 1, ..., N_y$ und $p_y$ dem Kanalabstand in y,
   - einen Pufferspeicher (50), in dem die von jedem der $2 \cdot (N_x + N_y) + 1$ Empfangskanäle für die Werte $l_0-1$, $l_0$, $l_0+1$ von k empfangenen Echographiesignale gespeichert werden,
   - eine zweite ($51_x$) bzw. dritte Interkorrelationsschaltung ($51_y$) zwischen einem auf dem mittleren Empfangskanal (0,0,0) empfangenen Signal $S_n(0,0,t)$ und dem auf dem Empfangskanal ($k_x p_x, 0, 0$) empfangenen nachfolgenden Signal $S_{n+1}(k_x, p_x, 0, 1+m\Delta T)$ bzw. dem auf dem Empfangskanal ($0, k_y p_y, 0$) mit $m = l_0-1$, $l_0$, $l_0+1$ empfangenen nachfolgenden Signal $S_{n+1}(0, k_y p_y, t+m\Delta T)$, woraus sich die drei Interkorrelationsfunktionen $C_x(k_x p_x, 0, (l_0-1)\Delta T)$, $C_x(k_x p_x, 0, l_0 \Delta T)$, $C_x(k_x p_x, 0, (l_0+1)\Delta T)$ bzw. $C_y(0, k_y p_y, (l_0-1)\Delta T)$, $C_y(0, k_y p_y, l_0 \Delta T)$, $C_y(0, k_y p_y, (lo+1)\Delta T)$ ergeben,
   - eine in x ($53_x$) bzw. y lineare Interpolationsschaltung ($53_y$), die auf der Grundlage der drei genannten Interkorrelationsfunktionen den Korrelationshöchstwert $C_x(k_x p_x)$ in x bzw. $C_y(k_y p_y)$ in y liefert,
   - eine zweite ($54_x$) bzw. dritte Höchstwertsuchschaltung ($54_y$), die ausgehend von den $2N_x + 1$ Werten von $k_x$ bzw. den $2N_y + 1$ Werten von $k_y$ den Wert $k_{x0}$ bzw. $k_{y0}$ liefern, für den $C_x(k_x p_x)$ bzw. $C_y(k_y p_y)$ am größten ist und die Komponenten in transversaler Richtung $V_x$ und $V_y$ die folgenden Gleichungen erfüllen:

$$V_x = k_{x0} p_x / T$$
$$V_y = k_{y0} p_y / T$$

   wobei T das Rekursionsintervall der Echographiesignale ist.

2. Gerät nach Anspruch 1,
dadurch gekennzeichnet, daß den genannten zweiten ($54_x$) und dritten Höchstwertsuchschaltungen ($54_y$) eine zweite ($54_x$) bzw. dritte ($54_y$) Interpolationsschaltung nachgeschaltet sind, die die Werte $k_{xmax}$ bzw. $k_{ymax}$ ergeben, für die Interkorrelationsfunktionen $C_x(k_x p_x)$ bzw. $C_y(k_y p_y)$ am größten sind und die transversalen Komponenten die folgenden Gleichungen erfüllen:

$$V_x = k_{xmax} p_x / T$$
$$V_y = k_{ymax} p_y / T$$

3. Gerät nach Anspruch 1 oder 2,
dadurch gekennzeichnet, daß die erste ($51_z$), zweite ($51_x$) und dritte ($51_y$) Interkorrelationsschaltung 1-Bit-Korrelatoren sind.

4. Gerät nach den Ansprüchen 2 und 3,
dadurch gekennzeichnet, daß die erste ($54_x$) und zweite Interpolationsschaltung ($54_y$) eine Linearinterpolation ausführen.

**5.** Gerät nach einem der vorangegangenen Ansprüche von 1 bis 4,
dadurch gekennzeichnet, daß ein Transversalinterpolator (40) vor dem genannten Pufferspeicher (50) eine Reduktion der Abstände $p_x$ und $p_y$ um den Faktor n vornimmt.

**6.** Gerät nach einem der vorangegangenen Ansprüche von 1 bis 5,
dadurch gekennzeichnet, daß der Kanalerzeugerschaltung (20) eine Schaltung (30) zur Eliminierung des Festechos nachgeschaltet ist.

$$e_n(k_x\, p_x, k_y\, p_y, t)$$

$$e'_n(k_x\, p_x, k_y\, p_y, t)$$

$$S_n(k_x\, p_x, k_y\, p_y, t)$$

FIG.1

50

$S_n(0,0t)$       $S_{n+1}(0,0,t)$

$51_z$   $\int$

$C_{nn+1}(0,0,k\Delta T)$

$52_z$   $\sum_n$

$C_z(0,0,k\Delta T)$

$I_0$   $Max(C_z)$   $53_z$

$C_z(0,0,m\,\Delta T)$

$m = I_0 - 1, I_0, I_0 + 1$

$54_z$

$k_{max}$

$V_z = C\,k_{max}\,\Delta T/2T$

FIG.2

$$S_n(0,0,t)$$

$$S_{n+1}(k_x\,p_x\,0, t+m\Delta T)$$
$$m = I_0-1, I_0, I_0+1$$

$$\int$$

$$C_{nn+1}(k_x\,p_x, 0, m\Delta T)$$

$$\Sigma_n$$

$$C_x(k_x\,p_x, 0, m\Delta T)$$

$$C_x(k_x\,p_x) \quad k_x = -1, 0, +1$$

$$\text{Max}(C_x)$$

$$k_{x_0}$$

$$C_x(l\,p_x)$$

$$l = k_{x_0}-1, k_{x_0}, k_{x_0}+1$$

$$k_x\,\text{max}$$

$$V_x = k_x\,\text{max}\; p_x / T$$

FIG.3